# EUROPEAN PATENT SPECIFICATION

(11) **EP 3 687 616 B1**
(45) Date of publication and mention of the grant of the patent: **19.06.2024**
(21) Application number: 18792715.7
(22) Date of filing: 01.10.2018
(51) Int. Cl.: A61M 25/00, A61M 25/01, A61B 5/20, A61B 5/03, A61F 2/00, A61M 39/22, A61M 39/24, A61B 5/00, A61F 2/04

(54) **INDWELLING CATHETER, CATHETER INTRODUCER MATING DEVICE AND SYSTEM COMPRISING BOTH**
DAUERKATHETER, KATHETEREINFÜHRUNGS- VORRICHTUNG UND SYSTEM BESTEHEND AUS DIESEN
CATHÉTER À DEMEURE, DISPOSITIF D'INTRODUCTION D'UN CATHÉTER ET UN SYSTÈME COMPRENANT LES DEUX

(30) Priority: 29.09.2017 US 201715721096; 16.10.2017 US 201715785405; 16.10.2017 US 201715785398; 16.10.2017 US 201715785403
(43) Date of publication of application: 05.08.2020
(73) Proprietor: Spinal Singularity, Inc., San Clemente, CA 92672 (US)
(72) Inventor: HERRERA, Derek, San Clemente, CA 92672 (US); KASALKO, Jeff, San Clemente, CA 92672 (US)
(74) Representative: Pizzoli, Antonio Mario
(86) International application number: PCT/US2018/053806
(87) International publication number: WO 2019/068104

(56) References cited:
- WO-A2-2016/118943
- WO-A2-2017/015351
- US-A- 4 932 938
- US-A1- 2017 156 838

## Description

### FIELD

This disclosure relates to a catheter system that can be used for bladder management, such as an extended-use catheter and devices that can connect to the extended use catheter in order to position it in a body and/or remove it from a body.

### BACKGROUND

Many people suffer from lower urinary tract dysfunction, also known as neurogenic bladder. Neurogenic bladder can be defined as impaired urinary function due to neurological injury or disease, such as spinal cord injury (SCI). Current methods for managing neurogenic bladder and other chronic urinary retention disorders are to drain the bladder using (a) intermittent catheterization (IC), or (b) indwelling Foley catheters. These methods, however, are associated with relatively high rates of urinary tract infection and genito-urinary (GU) injury, each of which diminishes a patient's quality of life. Furthermore, because some individuals with neurogenic bladder lack bladder sensation, and thus cannot accurately perceive bladder fullness, they are susceptible to bladder overfilling. This can result in urinary "accidents" and/or urinary reflux, and urinary reflux presents a risk of infection and tissue damage to the upper urinary tract.

To avoid these problems, individuals with neurogenic bladder on an IC program commonly rely on a timed catheterization schedule. This approach is imprecise and may lead to catheterization more frequently than necessary, which can increase the risk of infection and GU injury. Therefore, it is desirable to provide an improved urinary prosthesis that helps to alleviate one or all of the preceding problems.

Said problems are solved by means of a catheter system as defined in claim 1, while US 2017/156838 A1 and WO 2016/118943 A2 disclose known catheter systems, each comprising some features of claim 1. For example, US2017/156838 discloses a catheter system comprising: a catheter comprising a tube with a proximal end with an opening in communication with a catheter lumen and provided with a valve, a catheter mating device comprising a stem with an apparatus having a first, retracted configuration and a second, expanded configuration and being configured to be received in the proximal end of the catheter in its first, retracted configuration, and being configured to engage the proximal end of the catheter when in its second, expanded configuration, wherein a housing is connected to the proximal end of the stem.

### SUMMARY

The present disclosure relates to catheter systems for extended-use bladder management and controlling urinary function for humans or other animals. The disclosed systems may be used for fluid flow control for other bodily organs as well, such as kidneys, or draining abscesses or fluids from a body, and the description herein for bladder-control use is not limiting.

Disclosed is an extended-use catheter configured for being retained inside of the body. The catheter can be used, for example, in the human male urinary tract. When in use, it is preferably positioned fully inside of, and retained in, the urethra and bladder. In one embodiment, the catheter comprises an elongated tube having a wall with an outer surface, and a lumen through which fluid, such as urine, can pass. Positioned on, or formed as part of, the tube wall is a retainer portion with a cross-sectional area greater than the cross-sectional area of the outer surface of the tube wall. When the catheter is properly positioned in the urinary tract, the retainer portion is positioned in the bulbar urethra, where it aids in the proper positioning of the catheter and helps prevent the catheter from inadvertently moving forward or backward.

A catheter according to this disclosure could be periodically inserted, removed, and replaced by the user without medical assistance or the aid of another individual, which is convenient and saves time and medical expense. Such a catheter could remain in the body for days or weeks without being removed, which alleviates the problem of catheterization multiple times per day.

The catheter includes a valve, wherein the valve can be operated to: (a) allow fluid to exit the proximal end of the catheter, where it can exit the body, or (b) prevent the flow of fluid out of the proximal end of the catheter. The valve is most preferably a magnetic valve controlled from outside of the body using a wireless controller that generates a wireless signal to the valve to open or close.

A catheter mating device configured to engage and move the catheter is also disclosed and has a distal end and a proximal end. The distal end is configured to connect to the proximal end of the catheter, and includes an apparatus moveable between: (a) a first position, wherein the apparatus is retracted, and (b) a second position, wherein the apparatus is expanded. When in the first position, the apparatus is configured to fit into the lumen (or a mating chamber of the catheter) at the proximal end of the catheter. Once placed in the lumen, the apparatus can be moved to its second position, wherein the apparatus expands until it presses against and engages the wall of the lumen (or inner wall of the mating chamber). That connects the catheter mating device to the catheter, and the catheter can then be pushed into, or removed from a body structure, such as a bladder and urethra by, respectively, pushing or pulling the catheter mating device. Thus, the catheter mating device can be used to: (a) accurately place the catheter inside of a body, and/or (b) remove the catheter from a body.

The catheter may be configured to include one or more sensors, which may be on, inside of, or embedded in material forming the catheter, or partially or entirely within the lumen. The one or more sensors can be at any suitable location on the catheter, such as at a position where they are positioned in the bladder when the catheter is properly positioned in the lower urinary tract of a human male. The one or more sensors could collect any relevant data, such as fluid pressure in the bladder, pH level of fluid, volume of urine in the bladder, and/or amount of blood or bacteria in urine. The one or more sensors could communicate with other devices, such as CT scanners, ultrasound devices, x-ray machines, electronic data storage devices, computers, cell phones, the wireless controller, the catheter mating device and/or sensors placed in toilets. The data collected by the sensors could be stored, analyzed and/or transmitted by a device including software configured for these functions.

Because the catheter can remain in the body for long periods, a sensor on the catheter can gather and send data over the entire period the catheter is in the body, as opposed to gathering data only at a specific time, such as when a patent is at a doctor's office or hospital.

A catheter according to aspects of the invention could also include one or more antennas to communicate with the one or more sensors, and transmit data collected by the one or more sensors. The catheter could have a second lumen that includes one or more antennas and one or more catheters.

As used herein, the term "user" means any person able to insert and/or remove a catheter as disclosed herein, and includes a patient, doctor, caregiver, and nurse. "Patient" means a person that uses a catheter as disclosed herein in his/her body. "Lower urinary tract" refers collectively to the urinary bladder and urethra. "Extended use" means a catheter that can be used without having to remove it from the body more than once every two days or longer.

### BRIEF DESCRIPTION OF THE DRAWINGS

Figure 1 is a side view of an exemplary catheter, with an open view at its proximal end.
Figure 1A is a cross-sectional view of the tube of the catheter of Figure 1 taken along lines 1A-1A.
Figure 2 shows the catheter of Figure 1 positioned in the lower urinary tract of a human male.
Figure 3A is a side view of an exemplary embodiment of a catheter retainer portion according to aspects of the invention.
Figure 3B is a cross-sectional view of the catheter retainer portion of Figure 3A taken along lines 3A-3A.
Figure 3C is a side, perspective view of an exemplary embodiment of a catheter retainer portion according to aspects of the invention.
Figure 3D is a side, perspective view of an alternative exemplary embodiment of a catheter according to aspects of the invention that has a plurality of retainer portions.
Figure 3E is a side, perspective view of an exemplary embodiment of a catheter according to aspects of the invention that has a plurality of retainer portions
Figure 4 is a close-up, open view of the proximal end of the catheter of Figure 1 showing a valve.
Figures 4A-4C show the valve of Figure 4 and components of the valve.
Figure 5 is an exemplary embodiment of a wireless valve wireless controller.

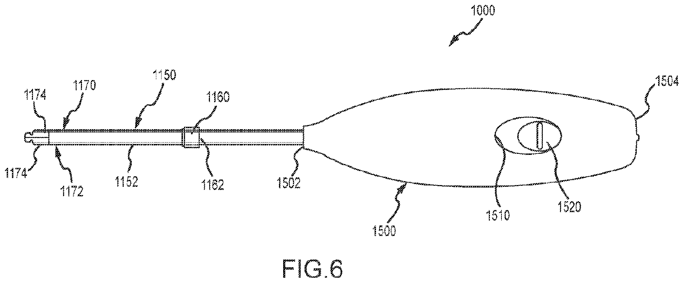

Figure 6 is a top view of an exemplary catheter mating device.
Figure 7 is an exploded view of the catheter mating device of Figure 6.
Figure 8 is a side, perspective, close-up view of the distal end of the stem of the catheter mating device of Figure 6.
Figures 8A and 8B are front views of the distal end of the stem of the catheter mating device of Figure 6.
Figures 8C and 8D are partial, side views of the distal end of the stem of the catheter mating device of Figure 6.
Figure 9 is a top view of the catheter mating device of Figure 6 (with the apparatus in its first, retracted position) being aligned with the catheter of Figure 1.
Figure 10 is another top view of catheter mating device of Figure 6 (with the apparatus in its first, retracted position) being aligned with the catheter of Figure 1.
Figure 11 is a side, open view of the catheter mating device of Figure 6 (with the apparatus in its first, retracted position) with its distal end positioned in the lumen at the proximal end of the catheter of Figure 1.
Figure 12 is a side view of an exemplary catheter that has one or more sensors, with an open view at its proximal end.
Figure 13 is a side view of an exemplary catheter having two lumens, with an open view at its proximal end.

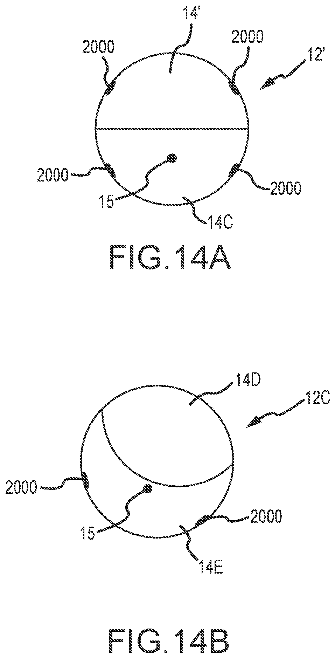

Figures 14A and 14B are cross-sectional views of exemplary catheters with two lumens.
Figure 15 shows the catheter mating device of Figure 6 (with the apparatus in its second, expanded position) engaged with the catheter of Figure 1.

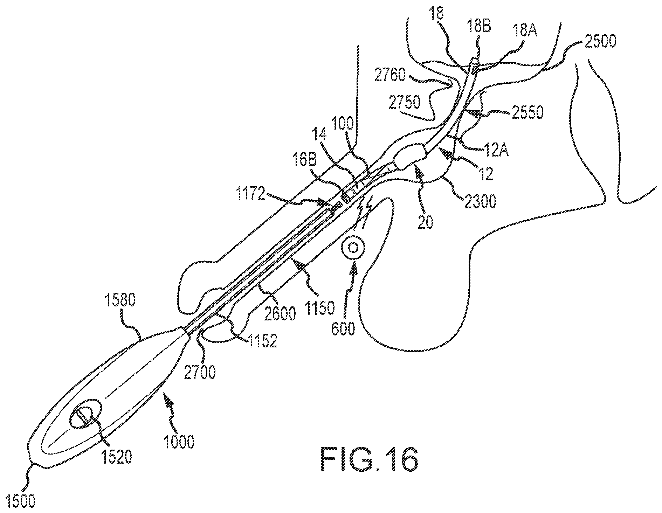

Figure 16 shows the catheter mating device of Figure 6 being aligned with the catheter of Figure 1 when the catheter is positioned in the lower urinary tract of a human male.

### DETAILED DESCRIPTION OF PREFERRED EMBODIMENTS

### Extended-Use Catheter

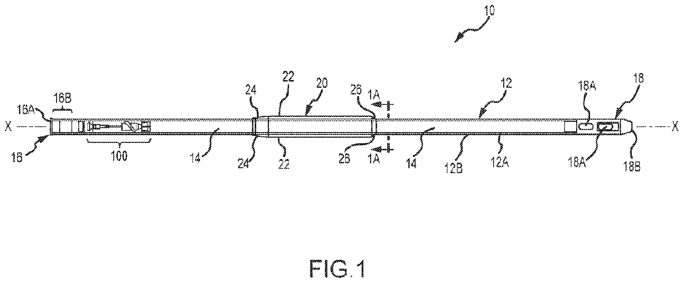

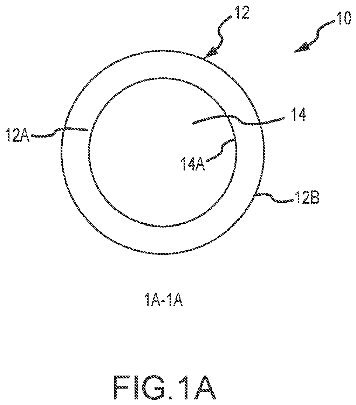

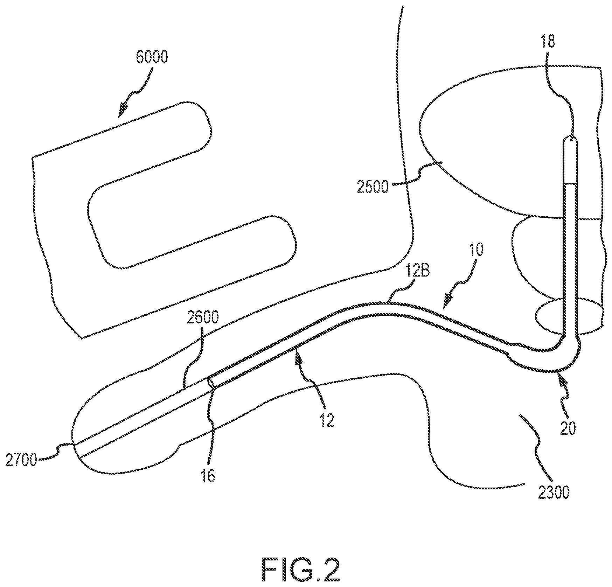

Turning now to the Figures, wherein the purpose is to describe preferred embodiments and not to limit the scope of the invention, Figure 1 shows an exemplary catheter 10 according to aspects of the invention. Catheter 10 is an extended use catheter, and can be shaped and sized to be introduced into, and retained in, the lower urinary tract of a human male. As shown in Figure 2, catheter 10 can extend from the bladder 2050 to a portion of the urethra 2600 distal to the prostate and distal to the bulbar urethra. Catheter 10 as shown in Figure 2 extends past the prostate and the external urinary sphincter. In a female anatomy, the retainer portion is preferably positioned within the urethra, between the internal urethral orifice and the external urethral orifice. The retainer portion can then be positioned such that it is in the urethra between the meatus and the external urethral sphincter. Catheter 10 is preferably fully internal to the body once properly installed.

Turning to Figures 1 and 1A, catheter 10 comprises a tube 12 with a wall 12A having an outer surface 12B, a lumen 14 with a lumen wall 14A, a valve 100, a retainer portion 20, a proximal end 16 possibly with a proximal tip 16A and an engagement chamber 16B, and a distal end 18 possibly with openings 18A and a distal tip 18B. The catheter tube 12, which is shown in cross section in Figure 1A, preferably has a circular cross-sectional shape, but can be of any shape suitable for the intended use of catheter 10. When used in the lower urinary tract of a human male, as shown in Figure 2, tube 12 preferably has an outer diameter (as measured across outer surface 12B) ranging from about 1 French (0.3 mm) to 20 French (6.6 mm), which is approximately the same as or less than the maximum expanded dimension of the urethra 2600. Wall 12 can have a hardness of any amount from: about 30 Shore A to 55 Shore D, or about 30 Shore A, or about 30 to 50 Shore A, or about 20 to 50 Shore A, although any suitable hardness for the intended use of catheter 10 would suffice.

Lumen 14 may have any suitable cross-sectional geometrical shape (e.g., circular (which is most preferred), oval, semi-circular, rectangular, triangular, trapezoidal, or crescent) and can have a cross-sectional surface area (which is the area inside of lumen wall 14A when viewed in cross section ) equivalent to the area of a 0.1 mm diameter circle to that of a 5.5 mm diameter circle. If the cross-sectional shape of lumen 14 is circular lumen 14 preferably has a diameter of any amount from: 0.1 mm to 5.5 mm. Lumen 14 may also comprise different cross-sectional areas along its length. For example, the cross-sectional area of the lumen may be greater where the valve 100 is positioned, and/or a greater cross-sectional area at the proximal end 16. Or, the lumen's cross-sectional area may be greater along its entire length distal to valve 100.

Tip 16A can have an outer diameter greater than the diameter of the outer wall 12B. For example, tip 16A may have a diameter of 0.5mm - 1 mm greater than outer wall 12B. The purpose of tip 16A having a slightly larger diameter is so a user can locate it by touch (e.g., by pressing against the skin and feeling the ridge at tip 16A) when tip 16A is positioned in the penile urethra.

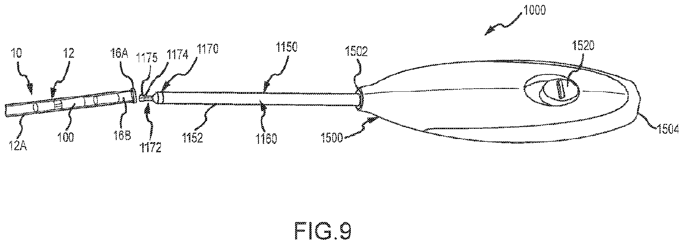

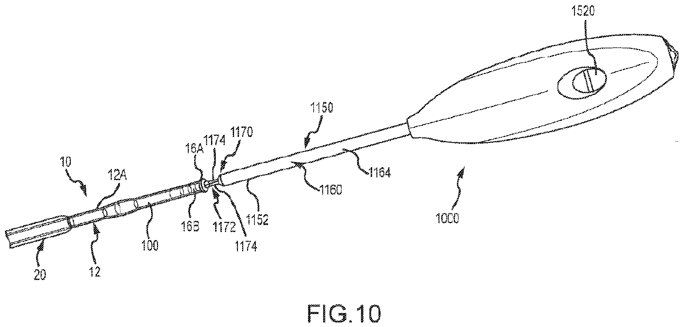

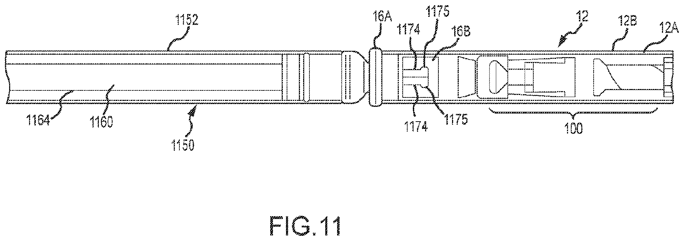

An engagement chamber 16B can be at or near the proximal end 16 of the catheter 10. The engagement chamber 16B can be located between the proximal tip 16A and the valve 100, or extend from proximal tip 16A to valve 100 or a position proximal valve 100. The engagement chamber 16B includes a space configured to engage the apparatus 1172 as generally shown in Figures 9-11 and described below. The engagement chamber 16B can have an annular, cylindrical shape, with a circular cross-section although any suitable shape may be used. As shown, the engagement chamber 16B has a circular cross-sectional shape with a diameter preferably form 0.1 mm to 5.5 mm. The engagement chamber 16B may have a hardness greater than the hardness of tube 12. For example, the hardness may be of any hardness between 10 Shore A to 55 Shore D harder than tube 12, or 10 Shore A - 20 Shore A harder, or 20 Shore A - 50 Shore A harder, or 50 Shore A - 55 Shore D harder. A purpose for engagement chamber 16B being harder than tube 12 is to better secure apparatus 1172 in engagement chamber 16B. It is not, however, required that catheter 10 have a mating chamber 16B. For example, apparatus 1172 could engage lumen wall 14A or another structure.

As shown in Figures 1 and 2, distal end 18 and tip 18B are configured to enter the patient's body through the urethral orifice 2700, and into the urethra 2600 when catheter 10 is positioned inside the lower urinary tract. As shown in this embodiment, distal tip 18B is tapered, rounded and closed. The distal tip 18B can comprise a material with hardness greater than the hardness of wall 12A. Distal end 18 permits the inflow of bodily fluid, such as urine, from a bladder or other body part into lumen 14, which can be accomplished in any suitable manner. One or more (as shown, two) openings 18A permit bodily fluid, such as urine, to enter lumen 14. As shown, openings 18A are on opposite sides of tube 12, so if one opening 18A is blocked because it is positioned against body tissue, the other opening 18A should still be unblocked. However, there need only be one opening, or there could be more than two openings, and the openings could be of any suitable size, configuration or location so they allow fluid, such as urine from the bladder, to enter lumen 14.

When catheter 10 is positioned in the lower urinary tract of a human male, the one or more openings 18A are positioned in the bladder 2500, as shown in Figure 2.

### Retainer Portion

The retainer portion 20 is positioned in the bulbar urethra 2300 when catheter 10 is properly positioned in the lower urinary tract of a human male. Retainer portion 20 is configured to prevent the inadvertent migration of catheter 10 either forward or backward once catheter 10 is properly positioned in the body. If positioned in the bulbar urethra, the retainer portion 20 is blocked by the external sphincter to prevent inadvertent retrograde migration, and blocked by the penile portion of the urethra 2600 to prevent inadvertent ante grade migration. When sufficient pulling or pushing force is applied to catheter 10, retainer portion 20 compresses so that it can pass through the urethra 2600 when catheter 10 is being removed from, or being placed in, the lower urinary tract.

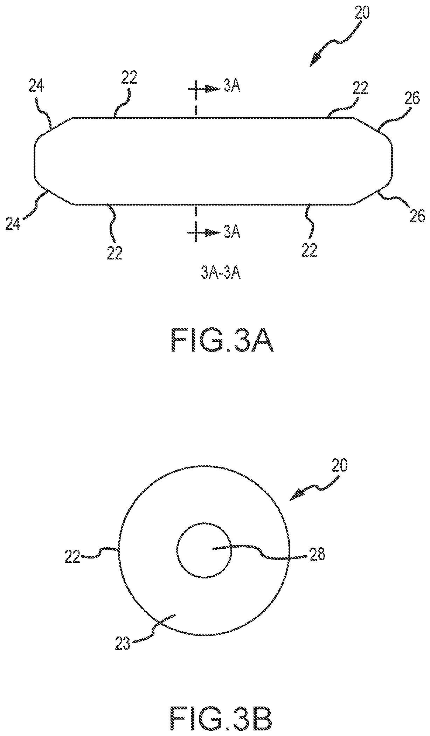

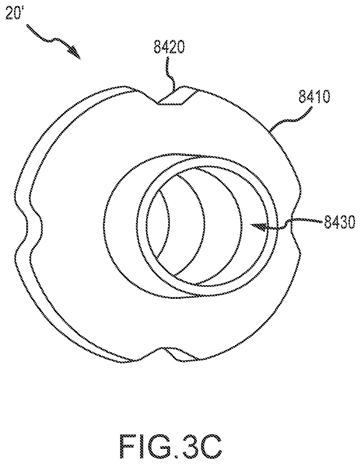

Retainer portion 20 is preferably formed: (a) over or as part of wall 12A of tube 12, or (b) as a separate part that has a passage, such as passage 28 shown in Figure 3B or passage 8430 shown in Figure 3C, wherein part of tube 12 is positioned snuggly in the passage. Each passage 28 and 8430 has a dimension configured to receive part of tube 12 and enable retainer portion 20 to fit snuggly on tube 12. Retainer portion 20 can comprise ribs, dimples, staples, or other structures on its outer surface to help retain it in the bulbar urethra or other body area.

As best seen in Figures 1 and 3A, the retainer portion 20 can comprise a top surface 22, proximal tapered surface 24, and a distal tapered surface 26. The proximal tapered surface 24 can be tapered from the top surface 22 to about the outer surface 12B of tube 12. The distal tapered surface 26 can be tapered from the top surface 22 to about the outer surface 12A of tube 12. The retainer portion has a length as measured along the longitudinal axis X of catheter 10. In one embodiment the retainer portion 20 has a total length of any amount from about: 1 cm to 10 cm, or 2 cm to 8 cm, or 3 cm to 7 cm, or 4 cm to 6 cm, and top surface 22 has a length of any amount from about: 1 cm to 10 cm, or about 10%, about 20%, about 30%, or about 40%, or about 50%, or about 60%, or about 70%, or about 80%, or about 90%, or about 95%, or any amount from about 5% to 95%, of the total length of retainer portion 20.

In an embodiment suitable for use in the lower urinary tract of a human male, the maximum cross-sectional area as measured inside of surface 22 (and including the cross-sectional area of passage 28) or surface 8410 (and including the cross-sectional area of passage 8430) is: (a) greater than the cross-sectional area of the external sphincter, (b) greater than the cross-sectional area of the penile urethra 2600, and (c) smaller than the cross-sectional dimension of the bulbar urethra 2300. The maximum cross-sectional area (as measured when retainer portion 20 is not being compressed) of the retainer portion 20 is 1.5 to two times larger, three times as large, four times as large, five times as large, six times as large, seven times as large, eight times as large, nine times as large, ten times as large, or any amount from at least 1.5 to ten times as large, as the cross-sectional area measured inside the outer surface 12B of tube 12. The maximum cross-sectional area (as measured when retainer portion 20 is not being compressed) may be any amount from: (24 mm)²π to (25 mm)²π, (4 mm)²π to (25 mm)²π, or (6mm)²π to (20 mm)²π, or (8 mm)²π to (16 mm)²π, or (10 mm)²π to (15 mm)²π, or (12 mm)²π to (15 mm)²π, or (5 mm)²π to (10 mm)²π. In one embodiment the top surface 22 has a circular cross-sectional shape and has a diameter of any amount from: 5 mm to 10 mm, or 5 mm to 7 mm, or 4 mm to 8 mm, or 6 mm to 15 mm, or 8 mm to 15 mm, or 6 mm to 20 mm, or 8 mm to 22 mm. The diameter of surface 12B (which is the outer diameter of tube 12) can be about 2.0 mm to 6.0 mm, or 4.6 to 6.0 mm, or any amount from: 1.5 mm to 6.5 mm.

Figure 3C shows alternate retainer portion 20' comprising one or more mounds 8410 and one or more grooves 8420 formed in its outer surface 8410. The retainer portion 20' can comprise alternating grooves 8420 and mounds 8410. For example, the retainer portion 20' can comprise four grooves 8420 and four mounds 8410 as shown in Figure 3C. The cross-sectional shape of the retainer portion could also symmetrical or asymmetrical, and be circular, cross-like, or any suitable shape for use with catheter 10.

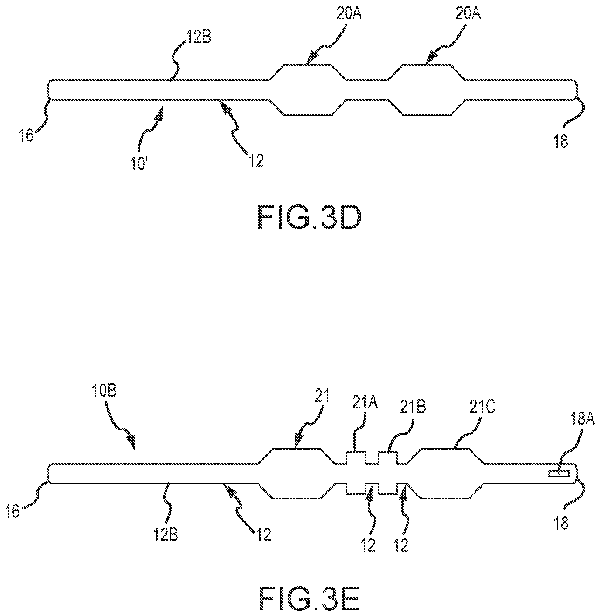

The catheter 10 may comprise two or more retainer portions 20A as shown in Figure 3D for catheter 10', or have any suitable number of retainer portions along wall 12, wherein the plurality of retainer portions could be of different shapes and/or sizes. For example, the catheter 10 can comprise a first retainer portion 20A and a second retainer portion 20A, wherein (if catheter 10 is being used in the lower urinary tract of a human male) both are positioned in the bulbar urethra when catheter 10 is positioned in a body. Retainer portions 20A would have respective lengths suitable for fitting into a body area such as the bulbar urethra, for their intended use.

Figure 3E shows a catheter 10B that has a plurality of retainer portions 21, 21A, 21B, and 21C of different shapes and sizes. The retainer portions would have suitable shapes and sizes for fitting into one or more body areas, such as the bulbar urethra.

Regardless of the structure or number of the retainer portion(s) utilized, any retainer portion can be comprised of solid material or include gas pockets. Each retainer portion should be atraumatic, and it preferably has a relatively soft, atraumatic surface. Retainer portion can be comprised of a flexible silicone and/or have a soft silicone surface coating. The retainer portion preferably has a durometer of any amount from: 1 to 40, or 5 to 30, or 10 to 25, or 15-25 Shore A. The retainer portion is preferably compressible. For example, the retainer portion can comprise a material that compresses to pass through cross-sectional areas smaller than the maximum cross-sectional area of the retainer portion. When used in the lower urinary tract of a human male, the retainer portion should compress to at least a dimension so that it can be pushed or pulled through the maximum expanded dimension of urethra 2600, which could be about 6 mm to 12 mm, or about 10 mm. The retainer portion expands once it is no longer restricted. When used in the lower urinary tract of a human male, the retainer portion is compressed when it is pulled through the urethra 2600 and expands once it enters the bulbar urethra 2300.

In some embodiments, the retainer portion may be compressed to about 80%, 75%, 70%, 60%, 50%, 40%, 30%, 25%, 20%, 10%, or any amount from about 10% - 80% of the maximum cross-sectional area. The retainer portion 20 is compressed to any such amount when a total force is applied substantially equally to the outer surface of the retainer portion at the maximum cross-sectional area in an amount of 907.19g (2 lbs.), 1360.78g (3 lbs.), 1814.37g (4 lbs.), 2267.96g (5 lbs.), 2721.55g (6 lbs.), 3175.15g (7 lbs.), 3628.74g (8 lbs.), 4082.33g (9 lbs.), 4535.92g (10 lbs.), 4989.51g (11 lbs.), 5443.10g (12 lbs.), 5896.70g (13 lbs.), 6350.29g (14 lbs.), 6803.88g (15 lbs.), 7257.47g (16 lbs.), 7711.06g (17 lbs.), 8164.66g (18 lbs.), 8618.25g (19 lbs.), 9071.84g (20 lbs.), or any force within the range of: 453.59g - 4535.92g (1 - 10 lbs.), 907.19g - 9071.84g (2 - 20 lbs.), or 226.79g - 2267.96g (0.5 - 5.0 lbs.). The retainer portion 20 can be physically compressed to 1/2 or less of the maximum cross-sectional area when subjected to a compressive force evenly applied along the outer surface of the cross-sectional area of an amount from: 1360.78g - 2267.96g (3-5 lbs.), or 907.19g - 1814.37g (2-4 lbs.), or 907.19g - 2721.55g (2-6 lbs.), or 1814.37g - 2721.55g (4-6 lbs.), or 2267.96g - 4535.92g (5-10 lbs.), or 3175.15g - 4535.92g (7-10 lbs.), or 2267.96g - 9979.03g (5-22 lbs.).

### Valve

The valve 100 is configured to restrict, or allow, fluid flow from the bladder 2000 (or other body part) out of the proximal end 16 of catheter 10. In the embodiment shown, the valve 100 is located in lumen 14 between the proximal end 16 and the retainer portion 20, although the valve 100 can be positioned at any suitable location in the lumen 14, or at the distal end 18 or proximal end 16 of catheter 10, as long as the valve can open to allow, and close to prevent fluid flow out of proximal end 16. As shown in this embodiment, the valve 100 is a magnetic valve, and wireless controller 6000 (described below) can be placed on or near valve 100 from outside of the patient's body to operate valve 100.

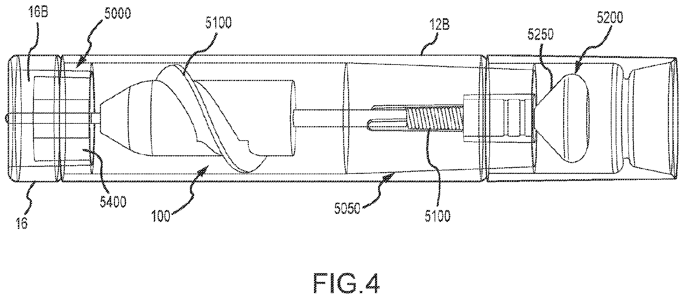

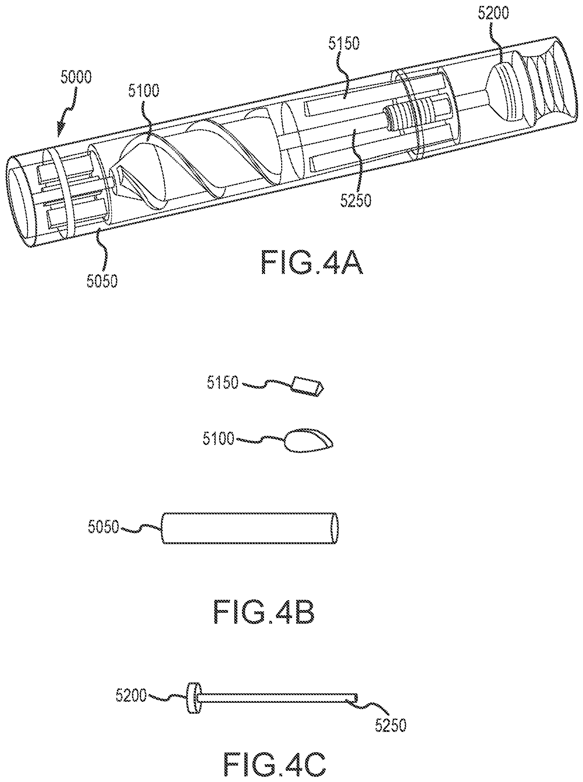

The valve 100 preferably comprises a cylindrical body. As shown in Figure 4, the valve 100 includes a housing 5050, a screw portion 5100, a valve magnet 5150, a valve tip 5200, a spindle 5250, and an alignment tube 5300. The housing 5050 can comprise a cylindrical body. The screw portion 5100 can comprise a threaded body. The valve tip 5200 can be connected to the spindle 5250. The valve tip 5200 can be configured to open and close the valve. For example, the valve tip 5200 can comprise a conical surface corresponding to a seating structure of the valve opening 5400.

The screw portion 5100 can be movable inside the housing 5050. The housing 5050 and the screw portion 5100 can be in a threaded connection. The magnet can be connected to the screw portion 5100 and the spindle 5250. By moving the magnet 5150, the valve 100 can open and close. For example, the magnet 5150 can be moved by using an wireless controller 600 (described below) to open and close the valve 100, such as by spinning magnet 5150, in order to activate the valve 100 and facilitate and/or control fluid flow. The valve 100 and the wireless controller 6000 can be configured to allow the user to increase or decrease the flow rate of the urine from the bladder 2500 by the wireless controller 600 signaling the valve 100.

If valve 100 is a magnetic valve, as shown in this embodiment, when it is operated it pumps fluid, rather than simply allowing fluid to flow as a result of fluid pressure in the bladder 2500 or other body structure in which distal end 18 is positioned. Using the bladder 2500 as an example, by pumping fluid the bladder is more completely emptied, which can lead to relieving the bladder fewer times over a given period of time. Alternatively, the valve could be any structure that can be operated to (a) prevent the passage of fluid out of proximal end 16, and (b) allow fluid to flow past proximal end 16.

### Catheter Mating Device

A system according to the invention comprises a catheter 10 and a catheter mating device 1000, wherein the catheter mating device 1000 and catheter 10 are each configured to connect to one another so that catheter 10 can be moved by moving the catheter mating device 1000. The catheter mating device 1000 can be used to place the catheter 10 into a patient's body, and to remove the catheter 10 from the patient's body. The catheter mating device 1000 comprises a stem 1150 that includes: (a) a tube 1152 having an outer surface (or external wall) 1154 and a lumen 1162, (b) an inner cylinder 1160 having a distal end 1160A and a proximal end 1160B connected to a control 1520, and (c) a distal end 1170 with apparatus 1172.

The apparatus 1172 is a structure that operates mechanically to connect the stem to the proximal end of the catheter, thus connecting the catheter mating device 100 to catheter 10. Apparatus 1172 has a first, retracted position, wherein it can fit inside of the proximal end 16 of tube 12, and a second, expanded position, wherein it engages proximal end 16 and connects catheter mating device 100 to catheter 10.

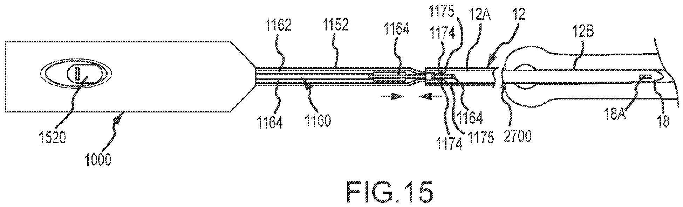

In this embodiment, the apparatus 1172 comprises tips 1174 that are configured to be positioned inside proximal end 16 (such as inside of engagement chamber 16B) when tips 1174 are in their first, retracted position and have a first distance between them (which may be zero distance because tips 1174 may touch when in the first, retracted position) as best seen in Figures 9-11. Tips 1174 can be moved to their second, expanded position in which they have a second distance between them that is larger than the first distance. In the second, expanded position, tips 1174 engage the inner wall of engagement chamber 16B, which connects catheter 10 to catheter mating device 1000. Once engaged, the catheter 10 can be moved through the urethra 2600, as shown best in Figure 15, by using the catheter mating device 1000 either to push and advance, or pull and retract, the catheter 10.

When in their second, expanded position, the tips 1174 (as measured when they are not restricted by a structure, such as the inner wall of mating chamber 16B or another structure), can have a maximum outer distance across them that is the same, or greater than, the diameter of mating chamber 16B (or other inner portion of catheter 10, such as lumen 14, because mating chamber 16B need not be used) in order for tips 1174 to create an interference fit against the inner wall of mating chamber 16B (or other structure). The tips 1174 can be comprised of any suitable material, such as a plastic, metal, or a thermoplastic elastomer.

In some embodiments, the tips 1174 at distal end 1170 form a tapered configuration such that they facilitate proper alignment of the tips 1774 and engagement chamber 16B so that tips 1774 can be received inside of chamber 16B (or other structure of catheter 10, such as lumen 14). Tips 1174 may have rounded end portions 1175 that assist in engaging proximal end 16.

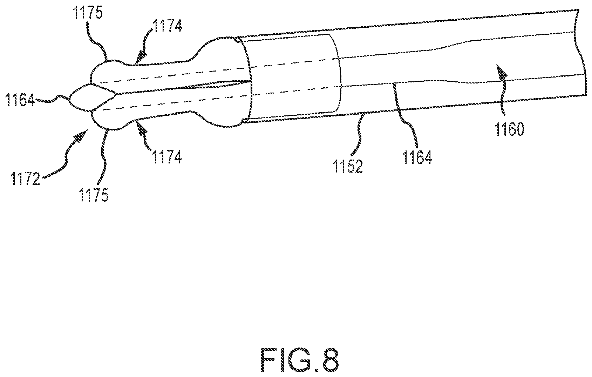

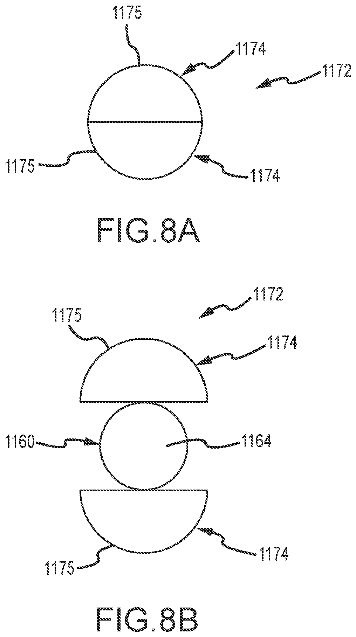

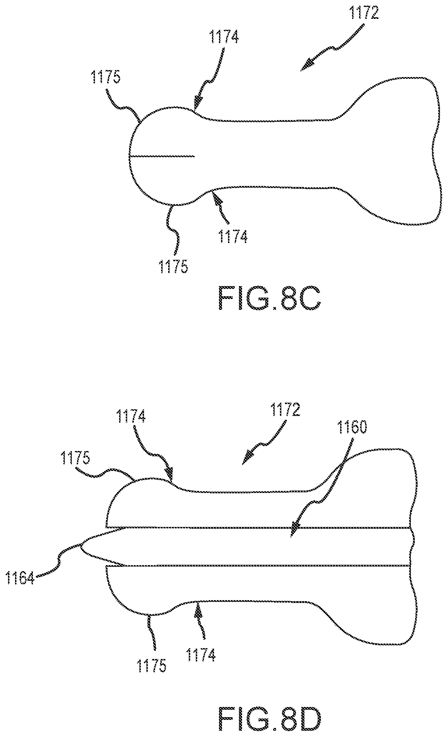

The catheter mating device 1000 in this embodiment comprises a cylinder 1160 positioned inside of outer tube 1152. The cylinder 1160 is operated to move from a retracted position to an extended position. When cylinder 1160 is in its extended position (as shown in Figures 8, 8B, and 8D), the cylinder 1160 moves between the tips 1174 and pushes them open to their second, extended position. When cylinder 1160 is in its retracted position (as shown in Figures 8, 8A, 8C, and 9-11) tips 1174 are in their first, retracted position. The cylinder 1160 may be comprised of any suitable material, such as ABS or PTFE, and preferably has a tapered distal end 1164.

A user may operate the catheter mating device 1000 by moving the control 1520 to its second position, which move the apparatus 1172 to its second, expanded position, and by moving the control 1520 to its first position, in which the apparatus 1172 either moves to, or is moved to its first, retracted position. In the embodiment shown, control 1520 is a slide switch directly or indirectly connected to cylinder 1160. When control 1520 is in its first position, the cylinder 1160 is in its first, retracted position. When control 1520 is moved to its second position, cylinder 1160 is moved to its second, extended position, wherein it moves between tips 1174 and pushes them open to their second, expanded position.

Although tips 1174 are shown as the apparatus 1172, other structures that can be mechanically expanded (as opposed to expanding utilizing gas or liquids) may be used. For example, there may more than two tips that are expanded in the manner described herein. Or, the tips or other structures may be expanded in any suitable manner. Alternatively, the tube 1152 of the stem 1150 could function as an outer sheath, and the apparatus could be attached to the cylinder 1160. In that example, the apparatus could be a metal mesh tube. Such an apparatus would be in its first, retracted position when contained inside of the tube 1152. The tube would be connected to the control 1520 and pulled back to expose the apparatus when the control 1520 is moved proximally (i.e., away from the catheter 10). That would permit the metal-mesh tube to automatically expand to its second, expanded position to engage proximal end 16. Or, utilizing the same structure, the cylinder 1160 including the apparatus could be connected to the control. In that case, a user could advance the cylinder 1160 and metal mesh tube out of the tube 1152, and the metal mesh tube would automatically expand to its second, expanded position. Alternatively, the stem could comprise an apparatus that is a flexible tube (comprised of rubber or flexible plastic) with one end of the tube connected to a first sleeve and another end connected to a second sleeve. The firs sleeve and second sleeve would be coaxial and at least one could move relative the other. A user could then manipulate one or both of the sleeves to compress the tube causing it to bulge, i.e., expand in the center, and engage the inner wall of the proximal end 16 (which could be engagement chamber 16B) of catheter 10.

The stem 1150 in this embodiment has a length configured to enable a user to place catheter 10 in the urinary tract using the catheter mating device 1000. For example, in this embodiment stem 1150 may have a lengths (a) greater than the length of the penile urethra of a patient, (b) less than or equal to the length as measured from the membranous portion to the urethral orifice of a patient, and/or (c) less than the length of the penile urethra of a patient. In some embodiments, the stem 1150 is about 10 cm to 26 cm in length. Stem 1150 can include a fluid opening 1162 that is configured to allow fluid flow from the catheter 10 to pass through the lumen 1164 of stem 1150 and out of the proximal end 1152A of tube 1152, where the fluid can be collected or disposed, or pass through housing 1500 to be collected or disposed.

### Housing

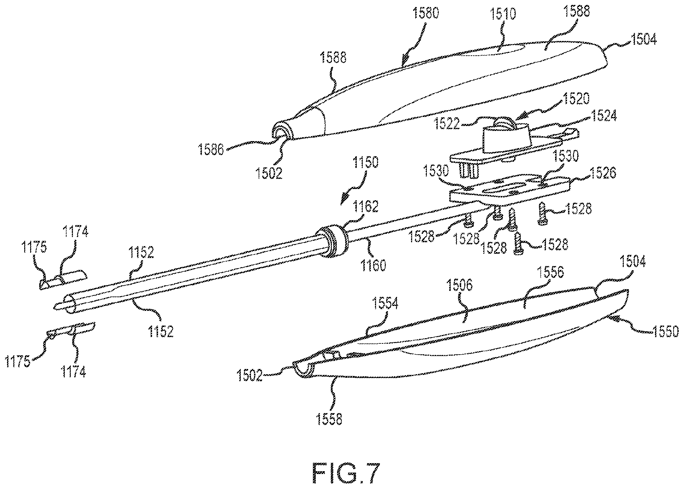

The catheter retainer portion 1000 has a handle (or housing) 1500 at its proximal end. Housing 1500 may house a portion of the stem 1150 and/or cylinder 1160, and includes a control 1520. The housing 1500 is comprised of any suitable material such as PVC or other plastic. Housing 1500 has body portion 1500A, which can be formed of two connected portions 1550 and 1580 as shown in Figure 7. Body portion 1500A has a distal end 1502, a proximal end 1504, and a cavity 1506. An opening 1510, which communicates with cavity 1506, is in surface 1588. A control 1520 is positioned in cavity 1506 and extends through opening 1510 where it can be accessed by a user.

As shown, control 1520 is a slide switch that can be moved from a first (proximal) position to a second (distal position). Control 1520 has a ridge 1522 that can be pushed by a user's finger, a body portion 1524 that extends through opening 1510, and a base 1526 positioned in cavity 1506. Fasteners 1528 extend through apertures 1530 to retain control in housing 1500.

In the embodiment shown, cylinder 1160 is connected to control 1520. A user can move switch 1520 in a distal direction to push cylinder 1160 to its extended position, wherein the cylinder 1160 moves the apparatus to its second, expanded position. A user can move switch 1520 in a proximal direction, wherein the cylinder 1160 moves to its retracted position away from the apparatus 1172 and the apparatus moves to its first, retracted position. Although a manual slide switch is described herein, control 1520 could be any structure that can operate to directly or indirectly move the apparatus to its second, expanded position.

The proximal end 1152A of tube 1152 is retained inside of cavity 1506. If fluid enters lumen (or passageway) 1164 it exits the proximal end 1152A, and can flow through housing 1500, where it exits opening 1504A at proximal end 1504. This allows for relatively easy collection or disposal of fluid from the body in which catheter 10 is positioned. In one embodiment, the stem 1150 has a length of about 10 cm to 26 cm.

### Materials

The catheter 10 and the stem 1170 of catheter mating device 1000 are, respectively, constructed in a shape and of a material that is conducive for their intended use. For example, the catheter 10 and stem 1170 may be constructed of any material or materials suitable for catheters used in the body (such as PVC, latex, silicone, polyurethane or any suitable blend of these materials).

### Packaging and Use

The catheter mating device 1000 and the catheter 10 can be carried in a sterilized pouch. A user may open the pouch comprising a catheter 10 and the catheter mating device 1000 and insert the catheter 10 into the lower urinary tract, and could use catheter mating device 1000 before inserting the catheter 10 into orifice 2700 of urethra 2600.

### Wireless controller

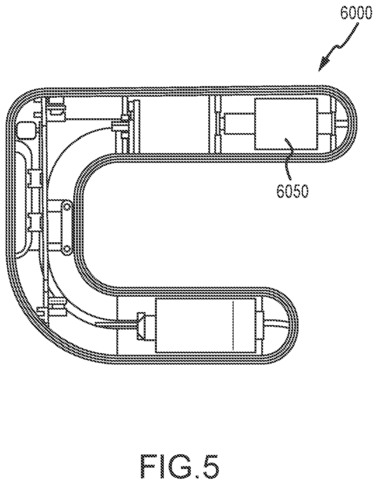

Urine in the bladder (or fluid from another area in the body) can be voided when the user utilizes an external wireless controller 6000 (shown in Figures 2 and 5) to operate the valve 100 and allow urine to travel through the lumen 14, past proximal end 16, and out of catheter 10. As shown in Figure 5, the wireless controller 6000 can comprise an wireless controller magnet 6050, a power source 6100, an electronic circuitry 6150, and one or more inputs 6200, 6250. In some embodiments, the one or more inputs 6200, 6250 can comprise a first input to open the valve 100 and a second input to close the valve 100. The wireless controller comprises two or more input modes, such as a close mode, an open mode, and an off mode. The valve 100 is closed (or off) when the wireless controller 6000 is in its close mode, and is open (or in operation) when the wireless controller is in its open mode. The valve 100 can remain closed or remain opened when the wireless controller 6000 is in its off mode. In some embodiments, the circuitry 6150 can comprise software configured to automate the process of controlling fluid flow from bladder. A user can place the wireless controller 6000 near the valve 100, e.g., on the skin of the patient between the scrotum and the shaft of the penis. The user may press the input 6200 to operate the magnet 6050.

### Sensors and Data Collection

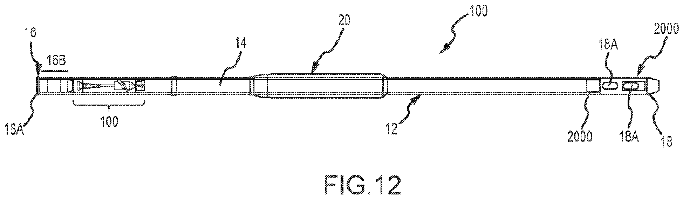

As shown in Figure 12, the catheter 10D can include one or more sensors 2000 configured to transmit data from the patient's body. In all other respects, catheter 10' is the same as catheter 10, described above. As used herein, any sensor used with a catheter according to the invention is referenced by numeral 2000. Thus, one or more sensors 2000 refers to a single sensor and a plurality of sensors. The data can comprise one or more of: fluid pressure of urine in the bladder and/or urethra, volume of fluid in the bladder, temperature of fluid in the bladder and/or urethra, acidity of fluid in the bladder and/or urethra, bacteria type and quantity of fluid in the bladder and/or urethra, chemical composition of fluid of fluid in the bladder and/or urethra, fluid flow during emptying of the bladder when valve 100 is open, or actuated. One or more sensors 2000 as described in this disclosure are of a type known to those skilled in the relevant art, although the claims are not limited to presently-known sensors. One or more sensors 2000 on or inside of catheter 10D would have basically consistent sensor location and extended sensors measurements within the body to better monitor patient conditions, without having sensors taken out of the body.

The one or more sensors 2000 can be placed at any suitable location on catheter 10D. For example, one or more sensors 2000 can be placed on or near the distal tip 18 of catheter 10D, in which case one or more sensors 2000 would be positioned in the bladder if catheter 10D is configured for use in the lower urinary tract of a human male. A catheter 10D could comprise different inner diameters can include a diameter in which a sensor 2000 would fit, such that the sensor is retained within lumen 14 without preventing fluid flow through the lumen. For example, a sensor 2000 could be distal to openings 18A. The outer wall 12A of the tube 12 would then prevent sensor 2000 from contacting body tissue or fluid. One or more sensors 2000 may be positioned on or in retainer portion 20.

In one embodiment, one or more sensors 2000 can determine the pressure of urine within the bladder and send a signal to a processor that sends the information to a computing device, or the one or more sensors 2000 could send data directly to the computing device. The computing device, which can be any device, such as a PC or other computer, cell phone, dedicated catheter device, the wireless controller, or the catheter mating device, can have software that determines whether and when fluid (such as urine) needs to be drained from the bladder. The computing device can notify the user in any manner to drain urine from his/her bladder. In some embodiments, the one or more sensors 2000 can be used to determine when urine has been sufficiently drained from the bladder, such as by determining that the pressure level within the bladder has dropped below a certain level. This information can be used to close the valve 100 and halt the flow of urine from leaving the bladder. In some embodiments, the one or more sensors 2000 can include acoustics to determine the volume of urine in the bladder. Different types of sensors can be placed in or on the catheter 10 to determine metrics related to the health of areas of the body, such as bladder health.

In some embodiments, a sensor 2000 can be configured to detect the flow rate of urine through the valve 100. For example, a catheter system 10D can comprise software configured to detect the flow rate of urine through the valve 100 by measuring the electric current draw of the valve motor by wireless controller 6000. The catheter system 10D can comprise an external computing device comprising a memory in wireless communication (either intermittently or continuously) with the electronic circuitry 6150 of wireless controller 6000 to record store and/or transmit data measured by one or more sensors 2000.

In some embodiments, one or more sensors 2000 can function without a power source. By constructing the sensor in a specific manner the external unit can observe changes in the resonant frequency characteristics.

The data collected by one or more sensors 2000 can be stored, analyzed, and/or transmitted via software resident on a device outside of the body in which one or more sensors 2000 is positioned. The software may utilize machine-learning algorithms to predict and interpret the measurements.

The sensor can be configured to change its mechanical properties (e.g. size or shape) based on pressure changes inside the bladder. The user can use an external device to detect changes in mechanical properties of the sensor by, for example, sending and/or receiving magnetic or electronic signals.

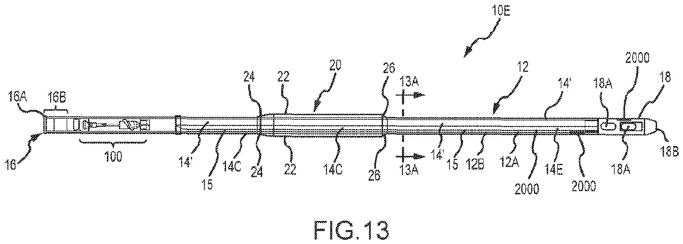

In another embodiment shown in Figure 13, catheter 10E has lumen 14 as described herein, and a second lumen 14'. In all other respects, catheter 10E is the same as previously described catheter 10D. Second lumen 14' can house one or more antennas 2002 that communicate in any suitable manner with the one or more sensors 2000 and with one or more external devices, such as a transducer or external computer device. Antennas 2002 can send data from one or more sensors 2000 to one or more external devices and signals from one or more external devices to any of the one or more sensors 2000. Antennas 2100 can have a power source 2004. One, some, or all of the one or more sensors 2000 may be partially or totally in second lumen 14'.

### Signal Transmission

One or more sensors 2000 can utilize basic wireless transmission protocol to send data to a computing device. This can be accomplished utilizing to Bluetooth, 802.11 WiFi, SONAR, UltraSound, MedRadio, or other wireless communications protocols. For example, the sensors can be configured to interface with CT, ultrasound, x-ray, and/or electronic data storage devices.

One or more sensors 2000 can comprise parts configured to interface and/or communicate with different products. For example, one or more sensors 2000 can be configured to interface with Amazon Echo ^{®} or Google Home ^{®}. One or more sensors 2000 could also be configured to interface with patient databases in hospitals or elsewhere. Information provided by a sensor 2000 may be formatted as desired. For example, analog data related to movement may be converted (using an analog to digital converter, for example) to a digital format, and subsequently formatted into a data packet including a data header followed by one or more data values.

Any amount of data can be transmitted in any manner. For example, data from one or more sensors 2000 can be transmitted to another device as the data is measured, or data can be stored (such as in a memory storage device) for a period of time before being transmitted to another device. In some cases, for example, it may be more efficient to transmit blocks of data at once rather than initiating communication with another device each time data is available. In other cases, a device may be out of range or otherwise unavailable to receive the data from one or more sensors 2000. The data can also be stored for any desired length of time, and/or until a particular event occurs. For example, the data could be stored by one or more sensors 2000 until the catheter mating device 1000 is connected to catheter 10' or 10", or until wireless controller 6000 is operated. Data could also be transmitted by one or more sensors 2000 to any device, such as the wireless controller 6000, catheter mating device 1000, or a cell phone. The data could be transmitted to such a device when the device is within a given range of one or more sensors 2000 (and hence a given range of the antenna(s) 2002, if utilized).

Data can also be deleted when a data record in a sensor 2000 exceeds a predetermined storage time, and/or the oldest data record is deleted first after a predetermined storage size limit has been reached.

### External Computer Device

The catheter system 1000 can comprise a computing device external to the patient's body. The external computing device may comprise a memory wirelessly connected to the electronic circuitry 6150 to receive operational parameters of the lower urinary tract. In some embodiments, the computing device can have a software which can be used to interpret data gathered the one or more sensors 2000. For example, one or more sensors 2000 may be a pressure sensor and the computing device can analyze data pressure received from the sensor 2000 be used to alert a user about when the user's bladder is likely to contract and void.

## Claims

1. A catheter system comprising:
(a) a catheter (10) comprising (i) a tube, the tube (12) having (A) a wall (12A) with an outer surface (12B), the outer surface (12B) having a first cross-sectional area, (B) a lumen (14), (C) a distal end (18) with one or more openings in communication with the lumen (14), and (D) a proximal end (16) with an opening in communication with the lumen (14), (ii) a valve (100) that is operated to be in (A) a closed configuration, wherein fluid does not flow out of the proximal end (16), or (B) an open configuration in which fluid does flow out of the proximal end (16); and (iii) a retainer portion (20) between the distal end (18) and the proximal end (16), the retainer portion (20) having a maximum cross-sectional area at least 1.5 to 10 times as large as the first cross-sectional area;
(b) a catheter mating device (1000) comprising: a stem (1150) having a proximal end (1160B) and a distal end (1160A); wherein the distal end (1160A) includes an apparatus (1172) having (i) a first, retracted configuration, and (ii) a second, expanded configuration; the apparatus (1172) being configured to be received in the proximal end (16) of the catheter when in its first, retracted configuration, and configured to engage the proximal end (16) of the catheter when in its second, expanded configuration; and
(c) a housing (1500) connected to the proximal end (1160B) of the stem (1150), wherein the housing (1500) includes a control (1520) having a first position and a second position, and the apparatus (1172) is in its first, retracted position when the control (1520) is in its first position, and the apparatus (1172) is in its second, extended position when the control (1520) is in its second position, wherein the apparatus (1172) comprises a plurality of tips (1174) moveable from the first, retracted position to the second, extended position.

2. The catheter system of claim 1, wherein the retainer portion (20) has a maximum cross-sectional area that is 1.5-4 times larger than the first cross-sectional area.

3. The catheter system of claim 1 or 2, wherein the retainer portion (20) has a length and the maximum cross-sectional area is at a center of the length.

4. The catheter system of claim 1, 2, or 3, wherein the maximum cross-sectional area is an area from: (4 mm)²π to (25 mm)²π.

5. The catheter system of claim 1, 2, or 3, wherein the retainer portion (20) is circular in cross-section at its maximum cross-sectional area, and has a diameter of 5 mm to 10 mm at the maximum cross-sectional area.

6. The catheter system of claim 1, 2, or 3, wherein the retainer portion (20) has an outer surface and can be physically compressed to 1/2 or less of the maximum cross-sectional area when subjected to a compressive force evenly applied along the outer surface of the cross-sectional area of an amount from: 1360.78g - 2267.96g (3-5 lbs.), or 907.19g - 1814.37g (2-4 lbs.), or 907.19g - 2721.55g (2-6 lbs.), or 1814.37g - 2721.55g (4-6 lbs.), or 2267.96g - 4535.92g (5-10 lbs.), or 3175.15g - 4535.92g (7-10 lbs.), or 2267.96g - 9979.03g (5-22 lbs.).

7. The catheter system of claim 1, 2, or 3, wherein the retainer portion (20) is configured to be compressed to have a maximum diameter of 0.3 mm to 8.0 mm when moved through a penile urethra and to have a maximum diameter of 4.0 mm to 15 mm when positioned in the bulbar urethra (2300).

8. The catheter system of claim 1, 2, or 3, wherein the retainer portion (20) includes a passage (28, 8430) therethrough and part of the tube (12) is positioned in the passage (28, 8430).

9. The catheter system of claim 1, 2, or 3 that further includes one or more sensors (2000) on or in the catheter (10).

10. The catheter system of claim 9, wherein the one or more sensors (2000) are configured to be positioned in a bladder when the catheter (10) is positioned in a lower urinary tract of a human male.

11. The catheter system of claim 10, wherein the one or more sensors (2000) are positioned at least partially in the lumen.

12. The catheter system of claim 1, wherein the control (1520) is a slide button on an outside surface of the housing (1500), the slide button moveable between the first position of the control and the second position of the control (1520).

13. The catheter system of claim 10 that further includes one or more antennas (2100), wherein the antennas (2100) are configured to transmit data received from the one or more sensors (2000).

## Patentansprüche

1. Kathetersystem, umfassend:
(a) einen Katheter (10), der umfasst: (i) einen Schlauch, wobei der Schlauch (12) aufweist: (A) eine Wand (12A) mit einer Außenfläche (12B), wobei die Außenfläche (12B) eine erste Querschnittsfläche aufweist, (B) ein Lumen (14), (C) ein distales Ende (18) mit einer oder mehreren Öffnungen, die mit dem Lumen (14) in Verbindung stehen, und (D) ein proximales Ende (16) mit einer Öffnung, die mit dem Lumen (14) in Verbindung steht, (ii) ein Ventil (100), das betätigt wird, um sich in (A) einer geschlossenen Konfiguration, in der kein Fluid aus dem proximalen Ende (16) fließt, oder (B) einer offenen Konfiguration, in der Fluid aus dem proximalen Ende (16) fließt, zu befinden; und (iii) einen Halteabschnitt (20) zwischen dem distalen Ende (18) und dem proximalen Ende (16), wobei der Halteabschnitt (20) eine maximale Querschnittsfläche aufweist, die mindestens 1,5-bis 10-mal so groß wie die erste Querschnittsfläche ist;
(b) eine Katheteraufnahmevorrichtung (1000), die umfasst: einen Schaft (1150) mit einem proximalen Ende (1160B) und einem distalen Ende (1160A); wobei das distale Ende (1160A) eine Vorrichtung (1172) umfasst, die aufweist: (i) eine erste, eingezogene Konfiguration und (ii) eine zweite, ausgefahrene Konfiguration; wobei die Vorrichtung (1172) konfiguriert ist, um in dem proximalen Ende (16) des Katheters aufgenommen zu sein, wenn sie sich in ihrer ersten, eingezogenen Konfiguration befindet, und konfiguriert ist, um in das proximale Ende (16) des Katheters einzugreifen, wenn sie sich in ihrer zweiten, ausgefahrenen Konfiguration befindet; und
(c) ein Gehäuse (1500), das mit dem proximalen Ende (1160B) des Schafts (1150) verbunden ist, wobei das Gehäuse (1500) eine Steuerung (1520) umfasst, die eine erste Position und eine zweite Position aufweist, und sich die Vorrichtung (1172) in ihrer ersten, eingezogenen Position befindet, wenn sich die Steuerung (1520) in ihrer ersten Position befindet, und sich die Vorrichtung (1172) in ihrer zweiten, ausgefahrenen Position befindet, wenn sich die Steuerung (1520) in ihrer zweiten Position befindet, wobei die Vorrichtung (1172) eine Vielzahl von Spitzen (1174) umfasst, die aus der ersten, eingezogenen Position in die zweite, ausgefahrene Position bewegbar sind.

2. Kathetersystem nach Anspruch 1, wobei der Halteabschnitt (20) eine maximale Querschnittsfläche aufweist, die 1,5- bis 4-mal größer als die erste Querschnittsfläche ist.

3. Kathetersystem nach Anspruch 1 oder 2, wobei der Halteabschnitt (20) eine Länge aufweist und sich die maximale Querschnittsfläche in einer Mitte der Länge befindet.

4. Kathetersystem nach Anspruch 1, 2 oder 3, wobei die maximale Querschnittsfläche eine Fläche von (4 mm)²π bis (25 mm)²π ist.

5. Kathetersystem nach Anspruch 1, 2 oder 3, wobei der Halteabschnitt (20) an seiner maximalen Querschnittsfläche einen kreisförmigen Querschnitt aufweist und an der maximalen Querschnittsfläche einen Durchmesser von 5 mm bis 10 mm aufweist.

6. Kathetersystem nach Anspruch 1, 2 oder 3, wobei der Halteabschnitt (20) eine Außenfläche aufweist und physisch auf 1/2 oder weniger der maximalen Querschnittsfläche zusammengedrückt werden kann, wenn er einer Druckkraft ausgesetzt wird, die gleichmäßig entlang der Außenfläche der Querschnittsfläche in einem Betrag von 1360,78 g - 2267,96 g (3-5 lbs.) oder 907,19 g - 1814,37 g (2-4 lbs.) oder 907,19 g - 2721,55 g (2-6 lbs.) oder 1814,37 g - 2721,55 g (4-6 lbs.) oder 2267,96 g - 4535,92 g (5-10 lbs.) oder 3175,15 g - 4535,92 g (7-10 lbs.) oder 2267,96 g - 9979,03 g (5-22 lbs.) ausgeübt wird.

7. Kathetersystem nach Anspruch 1, 2 oder 3, wobei der Halteabschnitt (20) konfiguriert ist, um so zusammengedrückt zu werden, dass er einen maximalen Durchmesser von 0,3 mm bis 8,0 mm aufweist, wenn er durch eine Penisurethra bewegt wird, und einen maximalen Durchmesser von 4,0 mm bis 15 mm aufweist, wenn er in der Bulbarurethra (2300) positioniert ist.

8. Kathetersystem nach Anspruch 1, 2 oder 3, wobei der Halteabschnitt (20) einen Durchgang (28, 8430) durch ihn hindurch umfasst und ein Teil des Schlauchs (12) in dem Durchgang (28, 8430) positioniert ist.

9. Kathetersystem nach Anspruch 1, 2 oder 3, das ferner einen oder mehrere Sensoren (2000) an oder in dem Katheter (10) umfasst.

10. Kathetersystem nach Anspruch 9, wobei der eine oder die mehreren Sensoren (2000) konfiguriert sind, um in einer Blase positioniert zu werden, wenn der Katheter (10) in einem unteren Harntrakt eines männlichen Menschen positioniert ist.

11. Kathetersystem nach Anspruch 10, wobei der eine oder die mehreren Sensoren (2000) mindestens teilweise in dem Lumen positioniert sind.

12. Kathetersystem nach Anspruch 1, wobei die Steuerung (1520) ein Schiebeknopf auf einer Außenfläche des Gehäuses (1500) ist, wobei der Schiebeknopf zwischen der ersten Position der Steuerung und der zweiten Position der Steuerung (1520) beweglich ist.

13. Kathetersystem nach Anspruch 10, das ferner eine oder mehrere Antennen (2100) umfasst, wobei die Antennen (2100) konfiguriert sind, um von dem einen oder den mehreren Sensoren (2000) empfangene Daten zu übertragen.

## Revendications

1. Système de cathéter comprenant :
(a) un cathéter (10) comprenant (i) un tube, le tube (12) ayant (A) une paroi (12A) avec une surface extérieure (12B), la surface extérieure (12B) ayant une première aire de section transversale, (B) une lumière (14), (C) une extrémité distale (18) avec une ou plusieurs ouvertures en communication avec la lumière (14), et (D) une extrémité proximale (16) avec une ouverture en communication avec la lumière (14), (ii) une valve (100) qui est actionnée pour être dans (A) une configuration fermée, dans lequel le fluide ne s'écoule pas hors de l'extrémité proximale (16), ou (B) une configuration ouverte dans laquelle le fluide s'écoule hors de l'extrémité proximale (16) ; et (iii) une partie de retenue (20) entre l'extrémité distale (18) et l'extrémité proximale (16), la partie de retenue (20) ayant une aire de section transversale maximale au moins 1,5 à 10 fois plus grande que la première aire de section transversale ;
(b) un dispositif d'accouplement de cathéter (1000) comprenant : une tige (1150) ayant une extrémité proximale (1160B) et une extrémité distale (1160A) ; dans lequel l'extrémité distale (1160A) comprend un appareil (1172) ayant (i) une première configuration rétractée, et (ii) une seconde configuration expansée ; l'appareil (1172) étant configuré pour être reçu dans l'extrémité proximale (16) du cathéter lorsqu'il est dans sa première configuration rétractée, et configuré pour engager l'extrémité proximale (16) du cathéter lorsqu'il est dans sa seconde configuration expansée ; et
(c) un boîtier (1500) relié à l'extrémité proximale (1160B) de la tige (1150), dans lequel le boîtier (1500) comprend une commande (1520) ayant une première position et une seconde position, et l'appareil (1172) est dans sa première position rétractée lorsque la commande (1520) est dans sa première position, et l'appareil (1172) est dans sa seconde position étendue lorsque la commande (1520) est dans sa seconde position, dans lequel l'appareil (1172) comprend une pluralité de pointes (1174) mobiles de la première position rétractée à la seconde position étendue.

2. Système de cathéter selon la revendication 1, dans lequel la partie de retenue (20) a une aire de section transversale maximale qui est 1,5 à 4 fois plus grande que la première aire de section transversale.

3. Système de cathéter selon la revendication 1 ou 2, dans lequel la partie de retenue (20) a une longueur et l'aire de section transversale maximale est au centre de la longueur.

4. Système de cathéter selon la revendication 1, 2 ou 3, dans lequel l'aire de section transversale maximale est une aire de : (4 mm)²π à (25 mm)²π.

5. Système de cathéter selon la revendication 1, 2 ou 3, dans lequel la partie de retenue (20) est circulaire en section transversale à son aire de section transversale maximale et a un diamètre de 5 mm à 10 mm à l'aire de section transversale maximale.

6. Système de cathéter selon la revendication 1, 2, ou 3, dans lequel la partie de retenue (20) a une surface extérieure et peut être physiquement comprimée à 1/2 ou moins de l'aire de section transversale maximale lorsqu'elle est soumise à une force de compression appliquée uniformément le long de la surface extérieure de l'aire de section transversale d'une quantité de : 1360,78 g à 2267,96 g (3 à 5 Ibs), ou 907,19 g à 1814,37 g (2 à 4 Ibs), ou 907,19 g à 2721,55 g (2 à 6 Ibs), ou 1814,37 g à 2721,55 g (4 à 6 Ibs), ou 2267,96 g à 4535,92 g (5 à 10 Ibs), ou 3175,15 g à 4535,92 g (7 à 10 Ibs), ou 2267,96 g à 9979,03 g (5 à 22 Ibs).

7. Système de cathéter selon la revendication 1, 2, ou 3, dans lequel la partie de retenue (20) est configurée pour être comprimée pour avoir un diamètre maximum de 0,3 mm à 8,0 mm lorsqu'elle est déplacée à travers un urètre pénien et pour avoir un diamètre maximum de 4,0 mm à 15 mm lorsqu'elle est positionnée dans l'urètre bulbaire (2300).

8. Système de cathéter selon la revendication 1, 2, ou 3, dans lequel la partie de retenue (20) comprend un passage (28, 8430) au travers et une partie du tube (12) est positionnée dans le passage (28, 8430).

9. Système de cathéter selon la revendication 1, 2, ou 3, qui comprend en outre un ou plusieurs capteurs (2000) sur ou dans le cathéter (10).

10. Système de cathéter selon la revendication 9, dans lequel le ou les capteurs (2000) sont configurés pour être positionnés dans une vessie lorsque le cathéter (10) est positionné dans un tractus urinaire inférieur d'un mâle humain.

11. Système de cathéter selon la revendication 10, dans lequel le ou les capteurs (2000) sont positionnés au moins partiellement dans la lumière.

12. Système de cathéter selon la revendication 1, dans lequel la commande (1520) est un bouton coulissant sur une surface extérieure du boîtier (1500), le bouton coulissant étant mobile entre la première position de la commande et la seconde position de la commande (1520).

13. Système de cathéter selon la revendication 10 qui comprend en outre une ou plusieurs antennes (2100), dans lequel les antennes (2100) sont configurées pour transmettre des données reçues du ou des capteurs (2000).
